# EUROPEAN PATENT APPLICATION

(11) **EP 4 759 360 A1**
(43) Date of publication of application: **17.06.2026**
(21) Application number: 24851120.6
(22) Date of filing: 09.08.2024
(51) Int. Cl.: A61P 7/02, C07D 519/00, C07C 269/06

(54) **PREPARATION METHOD FOR SMTP-7 DERIVATIVE AND INTERMEDIATE THEREOF**

(30) Priority: 10.08.2023 CN 202311003778
(71) Applicant: Shanghai Senhui Medicine Co., Ltd., Shanghai 201203 (CN); Shanghai Shengdi Pharmaceutical Co., Ltd, Shanghai 201210 (CN); Jiangsu Hengrui Pharmaceuticals Co., Ltd., Lianyungang, Jiangsu 222047 (CN)
(72) Inventor: ZHU, Lingjian, Shanghai 201203 (CN); YU, Xiuzhao, Shanghai 201203 (CN); HAN, Guoying, Shanghai 201203 (CN); CONG, Haixu, Shanghai 201203 (CN); HUANG, Jian, Shanghai 201203 (CN)
(74) Representative: Regimbeau
(86) International application number: PCT/CN2024/110961
(87) International publication number: WO 2025/031476

(57) **Abstract**

A preparation method for an SMTP-7 derivative and an intermediate thereof. The present disclosure relates to a preparation method for δ-deuterated-L-ornithine. The method achieves a high yield, has mild reaction conditions, and is suitable for industrial production.

## Description

### Technical Field

The present disclosure belongs to the field of medicine, and relates to a preparation method for an SMTP-7 derivative and an intermediate thereof.

### Background Art

SMTP-7 (TMS-007, *Stachybotrys microspore* triprenyl phenol-7) was isolated in 2000 from a specific mold (*Stachybotrys microspora*) found on deciduous leaves in Iriomote Island, Okinawa Prefecture. It is a small-molecule plasminogen activator with a structure similar to that of vitamin E. It has a novel mechanism of action for dissolving blood clots and is also believed to be capable of inhibiting local inflammation at the thrombus site. In addition, SMTP-7 also exhibits anti-tumour angiogenesis activity, antioxidant properties, and tissue regeneration-promoting activity.

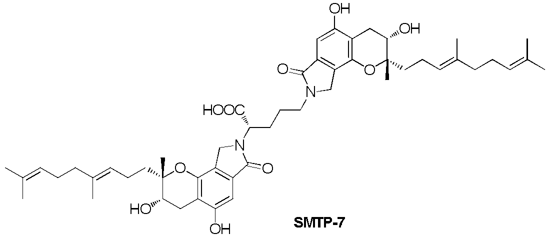

Plasminogen is the precursor of plasmin, which can be converted into plasmin upon activation. Plasmin is a protease that can hydrolyse various proteins, including thrombus proteins. Upon binding to plasminogen, SMTP-7 changes the molecular conformation of plasminogen, making it more susceptible to activation by plasminogen activators. Thus, SMTP-7 itself does not have the function of activating plasminogen, but merely facilitates the activation process. This unique combined effect of SMTP-7 makes it a promising best-in-class thrombolytic drug for the treatment of acute ischemic stroke (AIS), with the potential to extend the therapeutic window compared with existing standard thrombolytic drugs. Although many antihypertensive, lipid-lowering, and anticoagulant drugs can prevent stroke, the only current therapeutic drug for ischemic stroke is recombinant tissue plasminogen activator (rt-PA, alteplase), whose main component is a glycoprotein containing 526 amino acids.

The SMTP molecule induces a conformational change in plasminogen, accelerating the rate at which plasminogen binds to fibrin and ultimately leading to activation into plasmin. In addition, SMTP induces the auto-cleavage of plasmin to generate human angiostatin-like fragments that inhibit angiogenesis. This activity is believed to be the mechanism underlying the anti-angiogenic and anti-tumour effects of the SMTP molecule. Furthermore, the increased activation of plasminogen induced by SMTP may regulate local extracellular proteolysis, thereby contributing to tissue remodelling, wound healing, and tissue regeneration.

WO 2022171151A provides an SMTP derivative, with a structure as represented by formula (I), 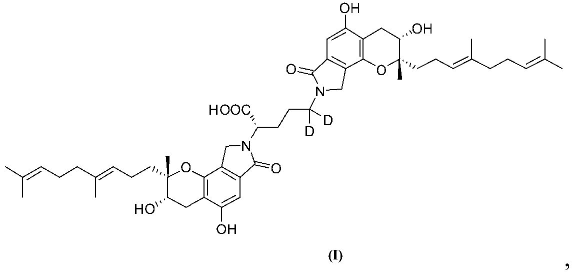 wherein δ-deuterated-L-ornithine is a key intermediate for preparing the compound as represented by formula (I). The existing preparation methods have drawbacks such as low yield and low deuteration rate, so a new method for preparing δ-deuterated-L-ornithine needs to be provided.

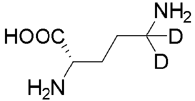

### Summary of the Invention

In one aspect, the present disclosure provides a method for preparing a compound as represented by formula (A-IV) or a salt thereof, comprising the step of preparing the compound as represented by formula (A-IV) from a compound as represented by formula (A-VI), wherein
R₁ is amino or amino protected by an amino protecting group,
R₂ is hydrogen or a carboxyl protecting group,

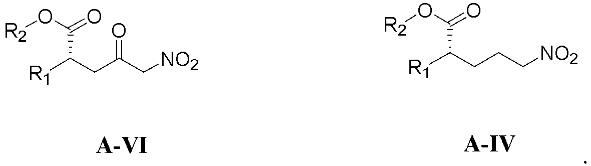

In another aspect, the present disclosure provides a method for preparing a compound as represented by formula (A-IV) or a salt thereof, comprising the step of preparing the compound as represented by formula (A-IV) from a compound as represented by formula (A-V), wherein
R₁ is amino or amino protected by an amino protecting group,
R₂ is hydrogen or a carboxyl protecting group,

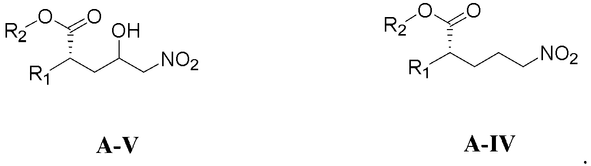

In some embodiments, the method comprises the steps of preparing a compound as represented by formula (A-V-1) from a compound as represented by formula (A-V), and preparing the compound as represented by formula (A-IV) from the compound as represented by formula (A-V-1), wherein
R₁ is amino or amino protected by an amino protecting group,
R₂ is hydrogen or a carboxyl protecting group,
R₃ is selected from formyl, acetyl, methanesulphonyl, trifluoroacetyl and benzoyl,

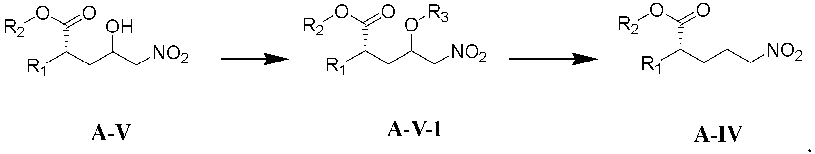

In some embodiments, the amino protecting group according to the present disclosure is selected from acetyl, methoxyacetyl, trifluoroacetyl, trichloroacetyl, pivaloyl, formyl, benzoyl, phthaloyl, 9-fluorenylmethoxycarbonyl, tert-butoxycarbonyl and benzyloxycarbonyl, such as tert-butoxycarbonyl and benzyloxycarbonyl.

In some embodiments, the carboxyl protecting group according to the present disclosure is selected from C₁-C₆ alkyl and C₆-C₁₀ aryl, such as methyl, ethyl and tert-butyl.

In some embodiments, R₁ is amino protected by an amino protecting group, and R₂ is a carboxyl protecting group.

In some embodiments, the reaction solvent is independently selected from one or more of ethyl acetate, isopropyl acetate, dimethylformamide, 1-methyl-2-pyrrolidone, tetrahydrofuran, methyltetrahydrofuran, dioxane, toluene, xylene, dimethyl sulphoxide, diethyl ether, isopropyl ether, methyl tert-butyl ether, acetonitrile, propionitrile, isopropanol, propanol, ethanol and methanol, such as one or more of isopropanol, propanol, ethanol and methanol.

In some embodiments, the reaction temperature for each step ranges from -20°C to 50°C, such as from -10°C to 30°C.

In another aspect, the present disclosure provides a method for preparing a compound as represented by formula (A-III) or a salt thereof, comprising the step of preparing the compound as represented by formula (A-III) from a compound as represented by formula (A-IV) in the presence of a deuterated reagent, wherein R₁ and R₂ are as defined above,

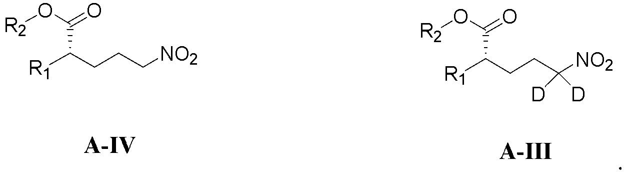

In some embodiments, the deuterated reagent is deuterated methanol or deuterium oxide.

In some embodiments, the reaction further contains a base, for example, an organic base or an inorganic base, such as at least one of triethylamine, trimethylamine, diisopropylethylamine, pyridine, or 4-dimethylaminopyridine.

In some embodiments, the method further comprises the step of preparing the compound as represented by formula (A-IV) or the salt thereof according to the present disclosure.

In another aspect, the present disclosure provides a method for preparing a compound as represented by formula (A-II) or a salt thereof, comprising the step of reducing a compound as represented by formula (A-III) to prepare the compound as represented by formula (A-II), wherein R₁ and R₂ are as defined above,

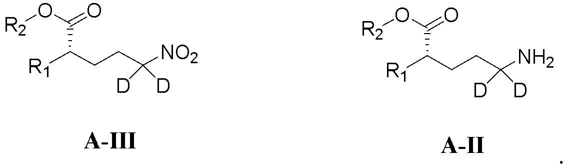

In some embodiments, the reduction reaction may be selected from iron powder reduction (iron powder combined with hydrochloric acid, acetic acid, or ammonium chloride), zinc powder reduction (zinc powder combined with hydrochloric acid, acetic acid, or ammonium chloride), metal catalyst-catalysed hydrogenation (using Raney nickel, palladium on carbon, etc. as catalysts and introducing hydrogen gas as a reducing agent), reduction with inorganic salt reducing agents (using sodium sulphide, sodium dithionite, sodium borohydride, etc. as reducing agents), and hydrazine hydrate reduction (using hydrazine hydrate as a reducing agent). For example, the reduction is carried out under zinc powder/acetic acid conditions.

In some embodiments, R₁ is amino protected by an amino protecting group, and R₂ is a carboxyl protecting group, and the method further comprises the step of deprotecting the compound as represented by formula (A-II) to prepare a compound as represented by formula (A-I) or a salt thereof,

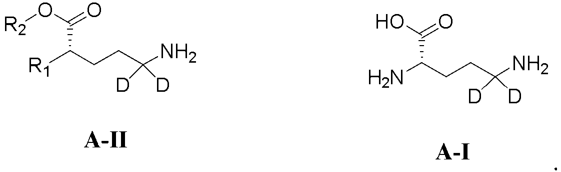

In some embodiments, the deprotection reaction is carried out in the presence of an acid, and the acid may be hydrochloric acid or trifluoroacetic acid.

In some embodiments, the method further comprises the step of preparing the compound as represented by formula (A-III) from the compound as represented by formula (A-IV) according to the present disclosure in the presence of a deuterated reagent.

In some embodiments, the method further comprises the step of preparing the compound as represented by formula (A-IV) or the salt thereof according to the present disclosure.

In another aspect, the present disclosure provides a method for preparing a compound as represented by formula (A-I) or a salt thereof, comprising the step of preparing the compound as represented by formula (A-I) from a compound as represented by formula (A-II), wherein R₁ is amino protected by an amino protecting group, and R₂ is a carboxyl protecting group,

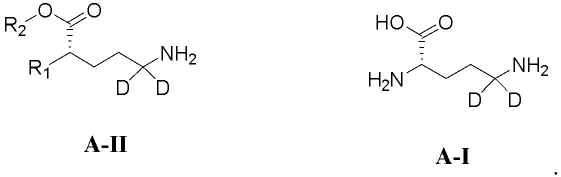

In some embodiments, the method comprises the steps of preparing a compound as represented by formula (A-II-1) from the compound as represented by formula (A-II), and preparing the compound as represented by formula (A-II-1) from the compound as represented by formula (A-II), wherein R₄ is amino protected by an amino protecting group,

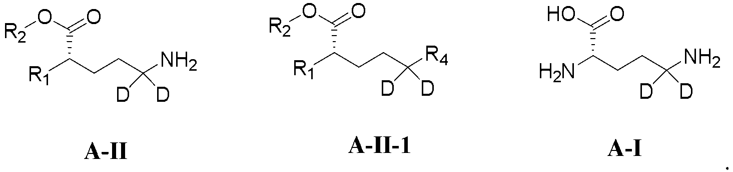

In another aspect, the present disclosure provides a method for preparing a compound as represented by formula (A-I) or a salt thereof, comprising 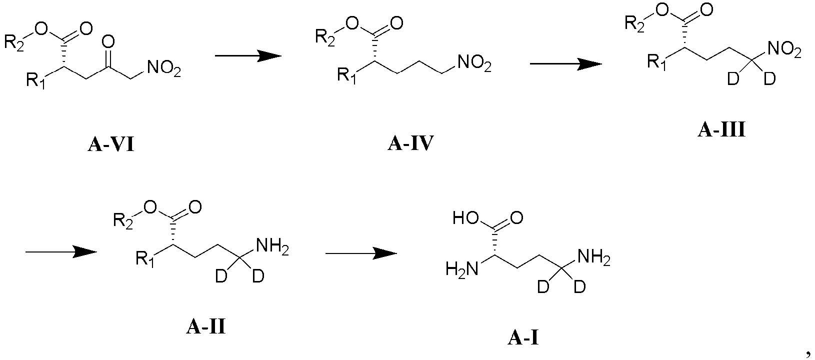 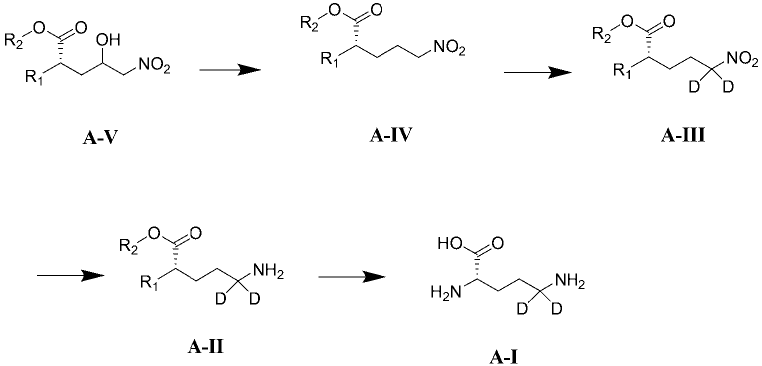 or 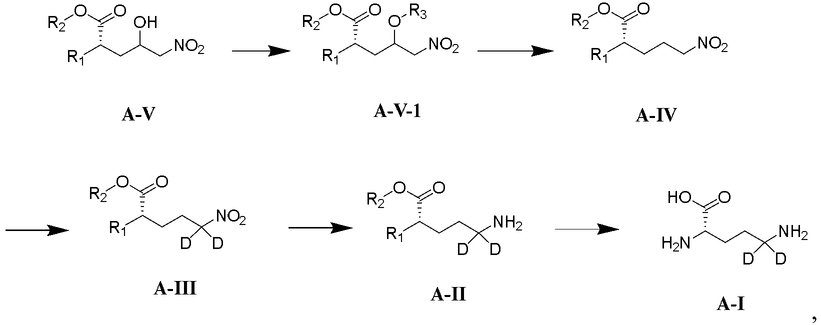 wherein R₁ is amino protected by an amino protecting group, and R₂ is a carboxyl protecting group.

In another aspect, the present disclosure provides a method for preparing a compound as represented by formula (A-I) or a salt thereof, comprising 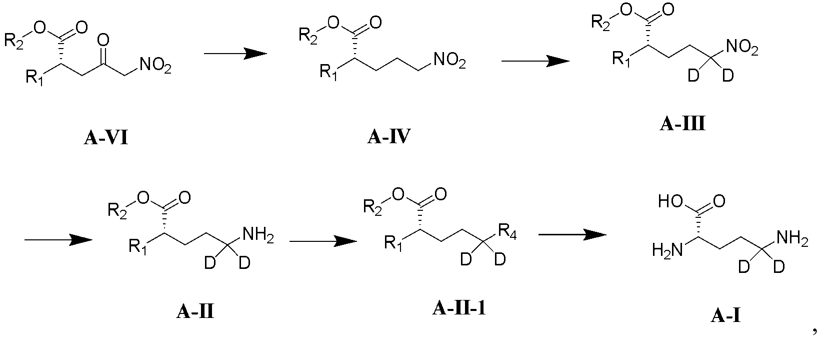 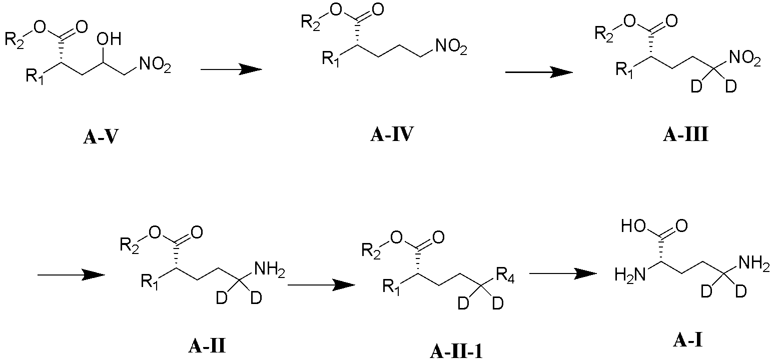 or 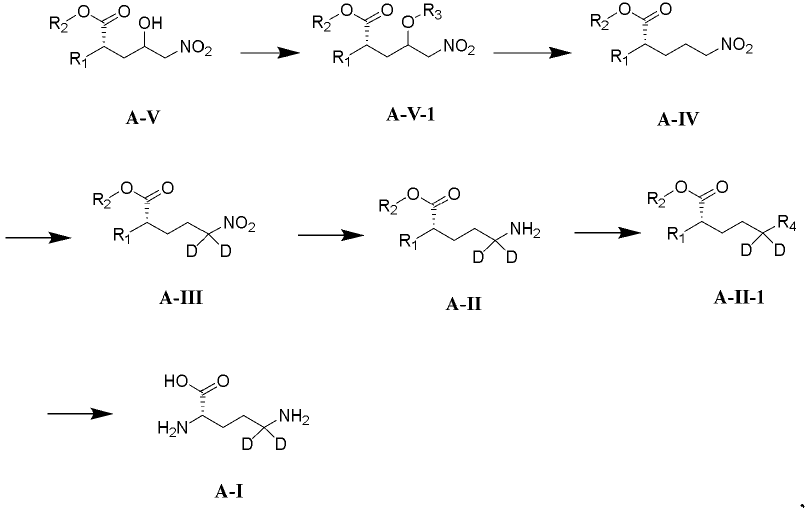 wherein R₁ is amino protected by an amino protecting group, R₂ is a carboxyl protecting group, and R₄ is amino protected by an amino protecting group.

In another aspect, the present disclosure provides a method for preparing a compound as represented by formula (I) or a pharmaceutically acceptable salt thereof, comprising one or more steps selected from the step of preparing the compound as represented by formula (A-I) or the salt thereof, the step of preparing the compound as represented by formula (A-II) or the salt thereof, the step of preparing the compound as represented by formula (A-III) or the salt thereof, and the step of preparing the compound as represented by formula (A-IV) or the salt thereof according to the present disclosure,

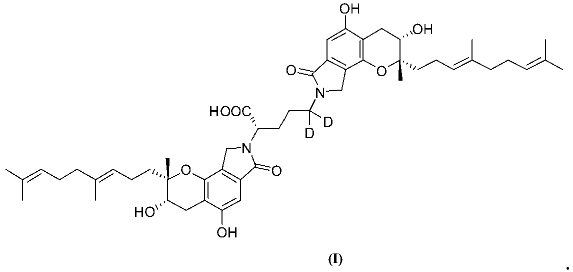

In some embodiments, the method further comprises the step of fermenting the compound as represented by formula (A-I) or the salt thereof to obtain the compound as represented by formula (I) or the pharmaceutically acceptable salt thereof.

The compound as represented by formula (I) or the pharmaceutically acceptable salt thereof may be prepared with reference to the method disclosed in WO 2022171151A, which is incorporated herein by reference in its entirety.

In some embodiments, each reaction solvent according to the present disclosure is independently selected from one or more of ethyl acetate, isopropyl acetate, dimethylformamide, 1-methyl-2-pyrrolidone, tetrahydrofuran, methyltetrahydrofuran, dioxane, toluene, xylene, dimethyl sulphoxide, diethyl ether, isopropyl ether, methyl tert-butyl ether, acetonitrile, propionitrile, isopropanol, propanol, ethanol, methanol and water.

In some embodiments, the preparation method of the present disclosure optionally further comprises a purification step, and the purification step comprises one or more of column chromatography, solvent trituration, and recrystallization.

The salts of the compounds according to the present disclosure may be inorganic acid salts and organic acid salts. The inorganic acid salts may include hydrochloride, sulphate, phosphate, hydrobromide, trifluoroacetate, etc., and the organic acid salts may include formate, acetate, sulphonate, optionally substituted alkyl sulphonate, succinate, maleate, tartrate, citrate, lactate, oxalate, gluconate, fumarate, malonate, malate, etc.

In another aspect, the present disclosure provides a compound as represented by formula (A-V-1) or a salt thereof, wherein
wherein
R₁ is amino or amino protected by an amino protecting group,
R₂ is hydrogen or a carboxyl protecting group,
R₃ is selected from formyl, acetyl, methanesulphonyl, trifluoroacetyl and benzoyl,

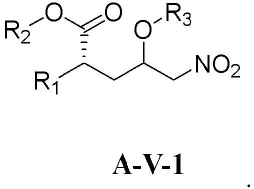

In another aspect, the present disclosure provides a compound as represented by formula (A-III), wherein R₁ is amino or amino protected by an amino protecting group, and R₂ is hydrogen or a carboxyl protecting group,

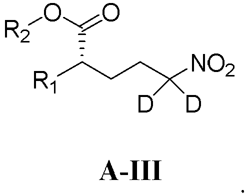

In another aspect, the present disclosure provides a compound as represented by formula (A-II) or a salt thereof, wherein R₁ is amino or amino protected by an amino protecting group, and R₂ is hydrogen or a carboxyl protecting group,

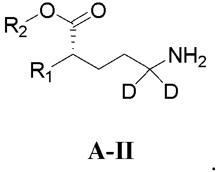

In another aspect, the present disclosure provides a compound as represented by formula (A-II-1) or a salt thereof, wherein R₁ is amino or amino protected by an amino protecting group, R₂ is hydrogen or a carboxyl protecting group, and R₄ is amino protected by an amino protecting group,

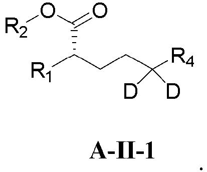

The preparation method for δ-deuterated-L-ornithine according to the present disclosure belongs to a chiral synthesis method, featuring high reaction yield, high product deuteration rate, mild reaction conditions, lower route cost, and greater suitability for industrial production.

In the preparation method according to the present disclosure, reactions connected by "→" denote that the product is obtained in a single step.

The "carboxyl protecting group" is a suitable group known in the art for carboxyl protection, see the carboxyl protecting group in the document ("Protective Groups in Organic Synthesis", 5Th Ed. T. W. Greene & P. G. M. Wuts). As an example, preferably, the carboxyl protecting group may be substituted or unsubstituted, linear or branched C₁₋₁₀ alkyl, substituted or unsubstituted, linear or branched C₂₋₁₀ alkenyl or alkynyl, substituted or unsubstituted cyclic C₃₋₈ alkyl, substituted or unsubstituted C₅₋₁₀ aryl or heteroaryl, or (C₁₋₈ alkyl or aryl)₃ silyl; preferably linear or branched C₁₋₆ alkyl, more preferably linear or branched C₁₋₄ alkyl, such as methyl, ethyl, allyl, prenyl, and trimethylsilylethyl.

The "hydroxyl protecting group" is a group known in the art for hydroxyl protection, see the hydroxyl protecting group in the document (Protective Groups in Organic Synthesis, 5Th Ed. T. W. Greene & P. G. M. Wuts). As an example, preferably, the hydroxyl protecting group may be (C1-10 alkyl or aryl)3 silyl, such as triethylsilyl, triisopropylsilyl, tert-butyldimethylsilyl, and tert-butyldiphenylsilyl; the hydroxyl protecting group may be C1-10 alkyl or substituted alkyl, such as methyl, tert-butyl, allyl, benzyl, methoxymethyl, ethoxyethyl, and 2-tetrahydropyranyl (THP); the hydroxyl protecting group may be (C1-10 alkyl or aryl)acyl, such as formyl, acetyl and benzoyl; the hydroxyl protecting group may be (C1-6 alkyl or C6-10 aryl)sulphonyl; the hydroxyl protecting group may also be (C1-6 alkoxy or C6-10 aryloxy)carbonyl, such as acetyl (Ac), 2-methoxyethoxymethyl ether (MEM), methoxymethyl ether (MOM), p-methoxybenzyl ether (PMB), and methylthiomethyl ether (MTM).

The "amino protecting group" is a group known in the art for amino protection, see the amino protecting group in the document (Protective Groups in Organic Synthesis, 5Th Ed. T. W. Greene & P. G. M. Wuts). As an example, the amino protecting group includes, but is not limited to a carbamate protecting group, such as 2-trimethylsilylethoxycarbonyl (Teoc), 1-methyl-1-(4-biphenyl)ethoxycarbonyl (Bpoc), tert-butoxycarbonyl (BOC), allyloxycarbonyl (Alloc), 9-fluorenylmethoxycarbonyl (Fmoc), and benzyloxycarbonyl (Cbz); an amide protecting group, such as formyl, acetyl, trichloroacetyl, benzoyl, and nitrophenylacetyl; a sulphonamide protecting group, such as 2-nitrobenzenesulphonyl; and imine and cyclic imine protecting groups, such as phthalimido and dithiasuccinoyl.

"Optional" or "optionally" means that the event or circumstance subsequently described may but not necessarily occur, and the description includes the occasion where the event or circumstance occurs or does not occur. For example, "heterocyclyl optionally substituted with alkyl" means that the alkyl may but not necessarily be present, and the description includes the case where the heterocyclyl is substituted with alkyl and the case where the heterocyclyl is not substituted with alkyl.

In the chemical structures of the compounds of the present disclosure, the bond " " indicates that the configuration is not specified, that is, if configurational isomers exist in the chemical structures, the bond " 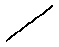 " may be " " 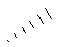 " "or " 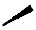 ", or contains both " 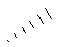 " and " 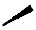 " configurations.

Although all the above structural formulas are depicted as certain isomeric forms for simplicity, the present disclosure may include all isomers, such as tautomers, rotamers, geometric isomers, diastereomers, racemates, and enantiomers.

### Detailed Description of Embodiments

The present disclosure will be explained in detail below in conjunction with specific examples to enable those skilled in the art to understand the present disclosure more comprehensively. These specific examples are only used to illustrate the technical solutions of the present disclosure and do not limit the present disclosure in any way.

### Example 1 Synthesis of δ-deuterated-L-ornithine

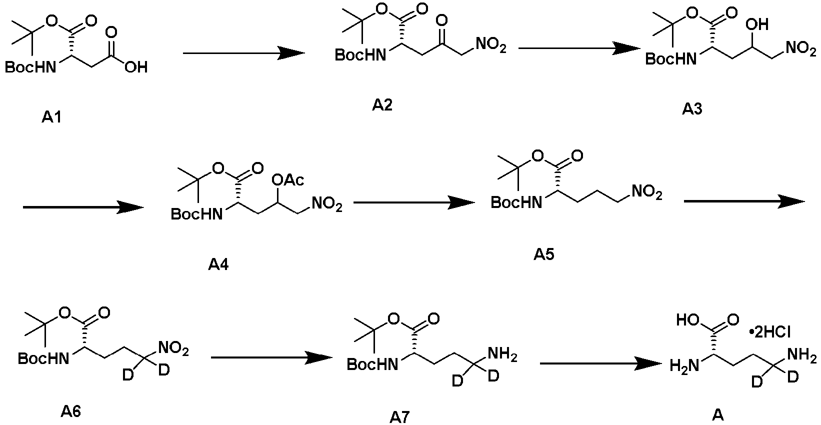

### Step 1: Synthesis of compound A2

Compound A1 (20 g, 69.2 mmol, 1.0 eq) and CDI (14.81 g, 91.35 mmol, 1.32 eq) were dissolved in THF (200 ml), and CH₃NO₂ (21.1 g, 346 mmol, 5.0 eq) was added under nitrogen atmosphere. The mixture was cooled to below 10°C in an ice-water bath, and t-BuOK (1M in THF, 139 ml, 138.4 mmol, 2.0 eq) was added dropwise. After the dropwise addition, the mixture was warmed to room temperature to continue the reaction. After the reaction was completed, the reaction solution was adjusted to pH = 2 with 2M HCl in an ice-water bath, and extracted with 500 ml of water and 200 ml of EA. The aqueous phase was extracted with 20 ml of EA. The organic phases were combined, washed with 100 ml of water and 100 ml of saturated sodium chloride aqueous solution, dried thoroughly over anhydrous sodium sulphate, filtered, and used directly in the next step.
MS-ESI: m/z 355.2 [M+Na]⁺

### Step 2: Synthesis of compound A3

AcOH (1V, 23 ml) was added to the organic phase from the previous step to prepare a mixed solution. Under nitrogen atmosphere, the mixture was cooled to an internal temperature of 10°C in an ice-water bath and stirred continuously for 10 min. NaBH₄ (5.26 g, 138.4 mmol, 2.0 eq) was added in portions, and after the addition, the reaction was continued in an ice-water bath until the starting materials were completely depleted. In an ice-water bath, 200 ml of water was slowly added to the reaction solution to quench the reaction. The aqueous phase was extracted once with 20 ml of EA. To the combined organic phases was added 400 ml of saturated sodium bicarbonate aqueous solution and stirred for about 60 min. The aqueous phase was extracted with 20 ml of EA. The organic phases were combined, washed with 100 ml of water and 100 ml of saturated sodium chloride aqueous solution, dried thoroughly over anhydrous sodium sulphate, filtered, and used directly in the next step.
MS-ESI: m/z 335.2 [M+H]⁺

### Step 3: Synthesis of compound A4

The reaction solution from the previous step was placed in an ice-water bath and stirred continuously for 10 min under nitrogen atmosphere. Ac₂O (21.19 g, 207.6 mmol, 3.0 eq) and DMAP (844 mg, 6.92 mmol, 0.1 eq) were added sequentially, and after the addition, the ice-water bath was removed and the mixture was allowed to rise naturally to room temperature to carry out the reaction. After the reaction was completed, 200 ml of water was added to the reaction solution for phase separation. The aqueous phase was extracted with 20 ml of EA. To the combined organic phases was added 300 ml of saturated sodium bicarbonate aqueous solution and stirred for 60 min for phase separation. The aqueous phase was extracted with 20 ml of EA. The organic phases were combined, washed with water and saturated sodium chloride aqueous solution (100 ml), dried thoroughly over anhydrous sodium sulphate, filtered, and concentrated to approximately 200 ml.
MS-ESI: m/z 399.2 [M+Na]⁺

### Step 4: Synthesis of compound A5

The reaction solution from the previous step was placed in a dry ice/ethanol bath. 250 ml of EtOH was added under nitrogen atmosphere, and the mixture was cooled to an internal temperature of -10°C. NaBH₄ (7.89 g, 207.6 mmol, 3.0 eq) was added in portions, and the reaction was maintained at this temperature. Then, 2M hydrochloric acid was slowly added to adjust to pH=2, and 300 ml of water and 100 ml of EA were added for phase separation. The aqueous phase was extracted with 50 ml of EA. The organic phases were combined, washed with saturated sodium bicarbonate aqueous solution, then washed with water and saturated sodium chloride aqueous solution, dried over anhydrous sodium sulphate, and concentrated under reduced pressure. The residue was purified by normal-phase silica gel column chromatography, and the resulting product was thoroughly dried under vacuum to obtain **A5** (15.68 g, yield over four steps: 70%, purity: 97%, chiral purity: 99.85%). MS-ESI: m/z 341.1 [M+Na]⁺.
HNMR (DMSO, 400M): 1.35-1.42 (m, 18H), 1.51-1.78 (m, 2H), 1.85-1.98 (m, 2H), 3.75-3.85 (m, 1H), 4.59 (t, J = 6.4 Hz, 2 H), 7.20 (t, J = 6.4 Hz, 1 H).

### Step 5: Synthesis of compound A6

A 250 mL single-necked flask was charged with **A5** (24.0 g, 75.3 mmol) and equipped with a pre-dried Y-shaped tube. The system was purged with argon three times, and THF (24.0 mL, 1V) was added via a syringe with stirring. Then, deuterium oxide (27.4 mL, 1.507 mol) and TEA (10.5 mL, 75.3 mmol) were added sequentially via a syringe, and the system was purged with argon three times. The system was heated in an oil bath at 35°C until the reaction was completed. The reaction solution was cooled in an ice-water bath, 72 mL of super-dry ethyl acetate was added and stirred for 5 min, and then the mixture was allowed to stand. The lower deuterium oxide layer was removed using a long needle and discarded. The organic phase was concentrated under reduced pressure to a constant weight of 24.1 g and detected by HNMR (D%=86.61%). MS-ESI: m/z 343.1 [M+Na]⁺

The above-mentioned single-necked flask was equipped with a pre-vacuum-dried Y-shaped tube, and purged with argon three times. Deuterium oxide (34.3 mL, 1.884 mol) was added via a syringe with stirring, and then pyridine (24.0 mL, 1V) was added via a syringe, and the system was purged with argon three times. The system was heated in an oil bath at 40°C until the reaction was completed. The reaction solution was subjected to in-process control by HNMR (**D%=99.40%**) and cooled in an ice-water bath, 120 mL of super-dry ethyl acetate was added and stirred for 5 min, and then the mixture was allowed to stand. The lower deuterium oxide layer was removed using a long needle and recovered for reuse. The organic phase was transferred to a constant-pressure dropping funnel and added dropwise to pre-prepared 1 N hydrochloric acid (455 mL) cooled to below 10°C in an ice-water bath. The phases were separated, and the aqueous phase was extracted once with 100 mL of regular ethyl acetate. The organic phases were combined, washed sequentially with 100 mL of water and 100 mL of saturated sodium chloride aqueous solution, dried over anhydrous sodium sulphate (10 g) for 10 min, filtered, and concentrated under reduced pressure to constant weight. This afforded **A6** (23.6 g, D% = 99.36%) with chiral data (ee = 99.76%) and an HPLC purity of 97.6%.
MS-ESI: m/z 343.1 [M+Na]⁺
¹H NMR (400 MHz, DMSO-d6) δ 7.21-6.83 (m, 1 H), 3.84-3.71 (m, 1 H) , 1.98-1.86 (m, 2 H), 1.72-1.53 (m, 2 H), 1.36-1.13 (m, 18 H).

### Step 6: Synthesis of compound A7

A 500 mL three-necked flask was equipped with a nitrogen balloon, a thermometer, and a 250 mL constant-pressure dropping funnel. Acetic acid (126 mL) was added, and the system was purged with nitrogen three times. Zinc powder (34.4 g, 529.6 mmol) was added in three portions, with the addition completed within 5 min. The mixture was then purged with nitrogen three times again, followed by stirring at 25°C-30°C for 5 min. Subsequently, freshly prepared solution of **A6** (11.3 g, 35.3 mmol) in acetic acid (11.3 g of **A6** dissolved in 100 mL of acetic acid) was added dropwise, and the reaction was stirred at room temperature until completion. The system was filtered, and the filter cake was washed with 100 mL of acetic acid. The filtrate was concentrated under reduced pressure. To the residue were sequentially added 100 mL of dichloromethane, 100 mL of saturated sodium carbonate solution and 100 mL of saturated sodium chloride aqueous solution, followed by stirring at room temperature (24°C-31°C) for 20 min. The mixture was suction-filtered, and the filter cake was rinsed with 200 mL of dichloromethane. The organic phases were combined, dried over anhydrous sodium sulphate (10 g), filtered, and concentrated under reduced pressure to obtain a crude product, which was purified by silica gel column chromatography to obtain **A7** (8.096 g in total, yield: 79%).
MS-ESI: m/z 291.1 [M+H]⁺.

### Step 7: Synthesis of compound A

To a 250 mL three-necked flask was added 23.1 mL of water. The flask was cooled to below 10°C in an ice-water bath, and concentrated hydrochloric acid (23.1 mL) was added dropwise. Subsequently, **A7** (8.050 g, 27.7 mmol) was added in portions, and the mixture was purged with nitrogen three times. After stirring continuously below 10°C for 30 min, the mixture was heated to an internal temperature of 55°C to carry out the reaction. After the reaction was completed, the mixture was cooled to 30°C and concentrated under reduced pressure. 62 mL of acetonitrile was added, followed by stirring at room temperature. The mixture was filtered, and the filter cake was rinsed with 20 mL of acetonitrile. The filter cake was dried under reduced pressure to obtain **compound A** (5.461 g in total, yield: 95%, deuteration rate: 99.16%, CAD data (purity: 98.84%)).
MS-ESI: m/z 135.2 [M+H]⁺.
¹H NMR (400 MHz, D₂O) δ 3.96 (t, *J =* 6.0 Hz, 1 H), 1.98-1.84 (m, 2 H), 1.80-1.64 (m, 2 H)

### Example 2 Synthesis of δ-deuterated-L-ornithine

δ-Deuterated-L-ornithine dihydrochloride was prepared according to the method disclosed in WO 2022171151A. The purity determined by CAD was 97.25%, and the deuteration rate was 97.3%, which was relatively low.

### Example 3

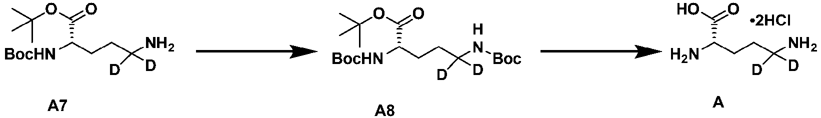

### Step 1: Synthesis of compound A8

Compound A7 (18.13 Kg, 62.4 mol) was dissolved in 150 L of dichloromethane. Di-tert-butyl dicarbonate (16.4 kg, 74.9 mol) and triethylamine (10.1 kg, 99.9 mol) were added, and the mixture was stirred at room temperature. The endpoint of the reaction was monitored by TLC. 140 L of water was added, and the mixture was stirred, filtered, and washed with 50 L of dichloromethane. The organic phases were combined, washed with 100 L of saturated sodium chloride aqueous solution, dried over anhydrous sodium sulphate, and concentrated. The residue was purified by silica gel column chromatography to obtain a crude product, which was slurried with petroleum ether to obtain compound A8 (20.83 kg in total, yield: 85.4%).
MS-ESI: m/z 389.2 [M+H]⁺.

### Step 2: Synthesis of compound A

Compound A8 (4.5 kg, 9.94 mol) was added to a mixed solution consisting of 22.5 L of acetonitrile, 13.5 L of water, and 13.5 L of concentrated hydrochloric acid. The mixture was heated to 55°C and reacted for 1 h, then stirred at room temperature overnight, concentrated, slurried with acetonitrile, filtered, washed, and dried under vacuum to obtain compound A (2.36 kg in total, yield: 98.9%, deuteration rate: 99.3%).

Since the present disclosure has been described in terms of its specific embodiments, certain modifications and equivalent variations would be obvious to those skilled in the art and are included within the scope of the present disclosure.

## Claims

1. A method for preparing a compound as represented by formula (A-IV) or a salt thereof, **characterised by** comprising the step of preparing the compound as represented by formula (A-IV) from a compound as represented by formula (A-VI), wherein
R₁ is amino or amino protected by an amino protecting group,
R₂ is hydrogen or a carboxyl protecting group,

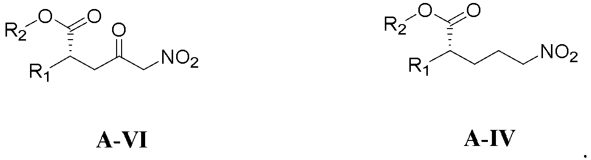

2. A method for preparing a compound as represented by formula (A-IV) or a salt thereof, **characterised by** comprising the step of preparing the compound as represented by formula (A-IV) from a compound as represented by formula (A-V), wherein
R₁ is amino or amino protected by an amino protecting group,
R₂ is hydrogen or a carboxyl protecting group,

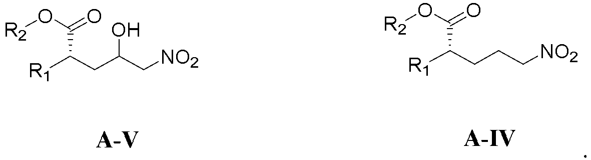

3. The preparation method according to claim 2, **characterised in that** the method comprises the steps of preparing a compound as represented by formula (A-V-1) from a compound as represented by formula (A-V), and preparing the compound as represented by formula (A-IV) from the compound as represented by formula (A-V-1), wherein
R₁ is amino or amino protected by an amino protecting group,
R₂ is hydrogen or a carboxyl protecting group,
R₃ is selected from formyl, acetyl, methanesulphonyl, trifluoroacetyl and benzoyl,

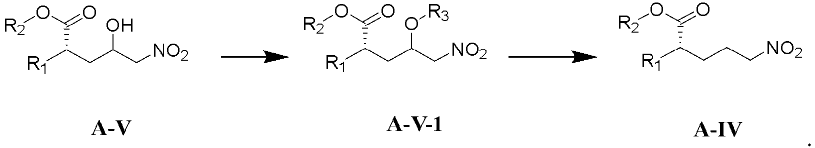

4. The preparation method according to any one of claims 1-3, **characterised in that** the amino protecting group is selected from acetyl, methoxyacetyl, trifluoroacetyl, trichloroacetyl, pivaloyl, formyl, benzoyl, phthaloyl, 9-fluorenylmethoxycarbonyl, tert-butoxycarbonyl and benzyloxycarbonyl, preferably tert-butoxycarbonyl and benzyloxycarbonyl.

5. The preparation method according to any one of claims 1-4, **characterised in that** the carboxyl protecting group is selected from C₁-C₆ alkyl and C₆-C₁₀ aryl, preferably methyl, ethyl and tert-butyl.

6. The preparation method according to any one of claims 1-5, **characterised in that** R₁ is amino protected by an amino protecting group, and R₂ is a carboxyl protecting group.

7. A method for preparing a compound as represented by formula (A-III) or a salt thereof, **characterised by** comprising the step of preparing the compound as represented by formula (A-III) from a compound as represented by formula (A-IV) in the presence of a deuterated reagent, wherein R₁ and R₂ are as defined in claim 1, and the deuterated reagent is preferably deuterated methanol or deuterium oxide,

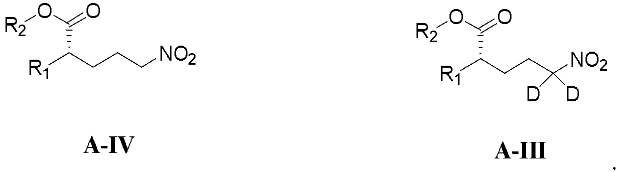

8. The preparation method according to claim 7, **characterised in that** the step further contains a base, preferably an organic base or an inorganic base, more preferably at least one of triethylamine, trimethylamine, diisopropylethylamine, pyridine, or 4-dimethylaminopyridine.

9. The preparation method according to claim 7 or 8, **characterised in that** the method further comprises the step of preparing the compound as represented by formula (A-IV) or the salt thereof according to any one of claims 1-6.

10. A method for preparing a compound as represented by formula (A-II) or a salt thereof, **characterised by** comprising the step of reducing a compound as represented by formula (A-III) to prepare the compound as represented by formula (A-II), wherein R₁ and R₂ are as defined in claim 1,

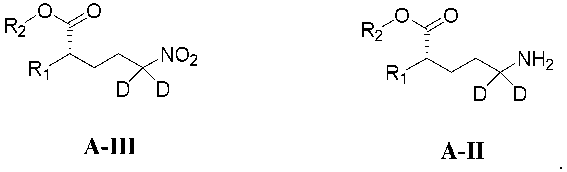

11. The preparation method according to claim 10, **characterised in that** R₁ is amino protected by an amino protecting group, and R₂ is a carboxyl protecting group, and the method further comprises the step of deprotecting the compound as represented by formula (A-II) to prepare a compound as represented by formula (A-I) or a salt thereof,

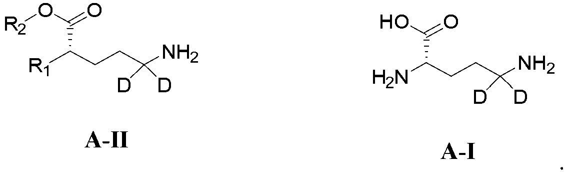

12. The preparation method according to claim 10 or 11, **characterised in that** the method further comprises the step of preparing the compound as represented by formula (A-III) or the salt thereof according to any one of claims 7-9.

13. A method for preparing a compound as represented by formula (A-I) or a salt thereof, **characterised by** comprising the step of preparing the compound as represented by formula (A-I) from a compound as represented by formula (A-II), wherein R₁ is amino protected by an amino protecting group, and R₂ is a carboxyl protecting group,

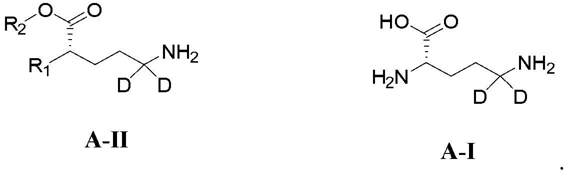

14. The preparation method according to claim 13, **characterised in that** the method comprises the steps of preparing a compound as represented by formula (A-II-1) from the compound as represented by formula (A-II), and preparing the compound as represented by formula (A-II-1) from the compound as represented by formula (A-II), wherein R₄ is amino protected by an amino protecting group,

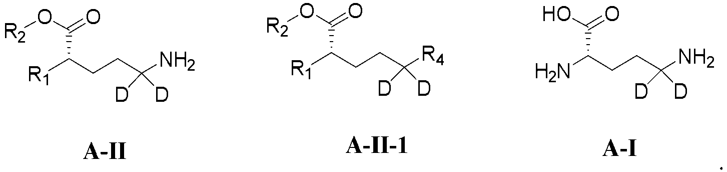

15. The preparation method according to claim 13 or 14, **characterised in that** the method further comprises the step of preparing the compound as represented by formula (A-II) or the salt thereof according to claim 10.

16. A method for preparing a compound as represented by formula (A-I) or a salt thereof, **characterised by** comprising 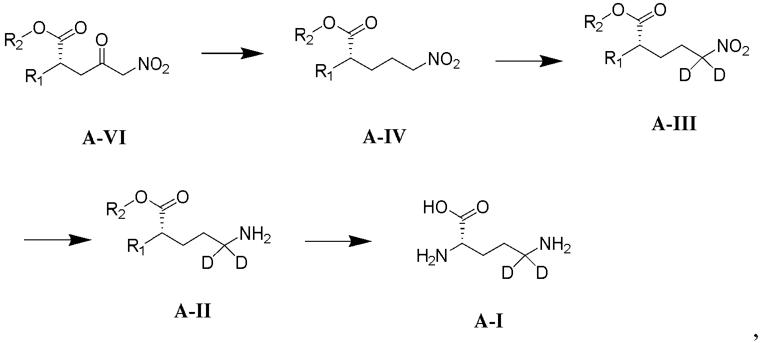 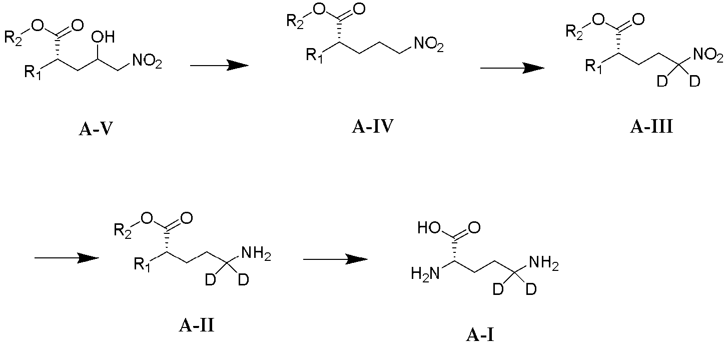 or 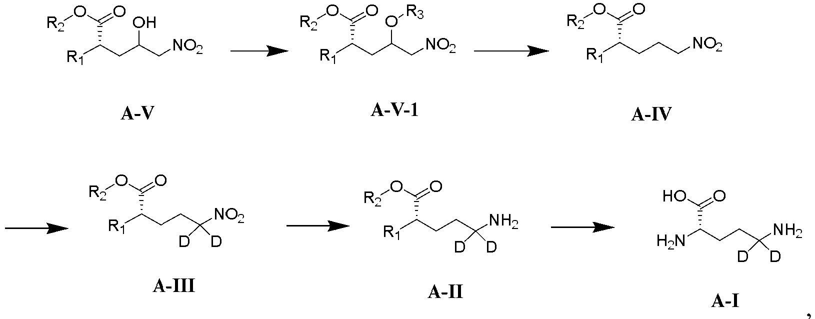 wherein R₁ is amino protected by an amino protecting group, and R₂ is a carboxyl protecting group.

17. A method for preparing a compound as represented by formula (A-I) or a salt thereof, **characterised by** comprising 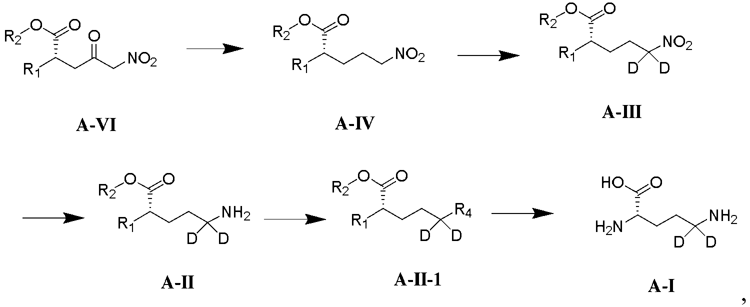 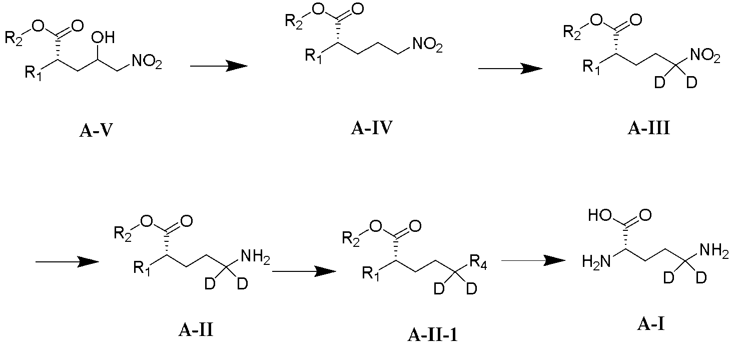 or 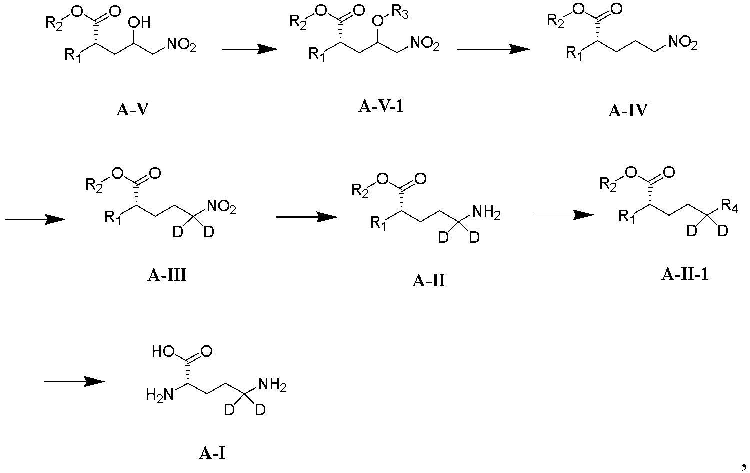 wherein R₁ is amino protected by an amino protecting group, R₂ is a carboxyl protecting group, and R₄ is amino protected by an amino protecting group.

18. A method for preparing a compound as represented by formula (I) or a pharmaceutically acceptable salt thereof, **characterised by** comprising one or more steps selected from the step of preparing the compound as represented by formula (A-I) or the salt thereof according to any one of claims 13-17, the step of preparing the compound as represented by formula (A-II) or the salt thereof according to any one of claims 10-12, the step of preparing the compound as represented by formula (A-III) or the salt thereof according to any one of claims 7-9, and the step of preparing the compound as represented by formula (A-IV) or the salt thereof according to any one of claims 1-6,

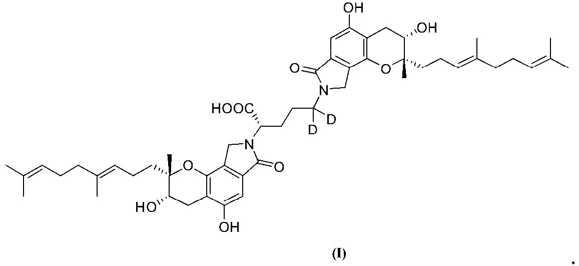

19. A compound as represented by formula (A-V-1) or a salt thereof, **characterised in that**
R₁ is amino or amino protected by an amino protecting group,
R₂ is hydrogen or a carboxyl protecting group,
R₃ is selected from formyl, acetyl, methanesulphonyl, trifluoroacetyl and benzoyl,

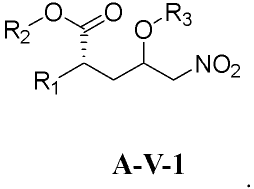

20. A compound as represented by formula (A-III) or a salt thereof, **characterised in that** R₁ is amino or amino protected by an amino protecting group, and R₂ is hydrogen or a carboxyl protecting group,

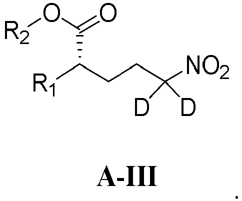

21. A compound as represented by formula (A-II) or a salt thereof, **characterised in that** R₁ is amino or amino protected by an amino protecting group, and R₂ is hydrogen or a carboxyl protecting group,

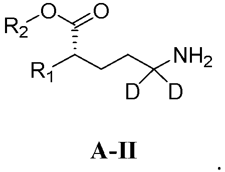

22. A compound as represented by formula (A-II-1) or a salt thereof, **characterised in that** R₁ is amino or amino protected by an amino protecting group, R₂ is hydrogen or a carboxyl protecting group, and R₄ is amino protected by an amino protecting group,
